# FASCICULE DE BREVET EUROPEEN

(11) **EP 0 371 838 B1**
(45) Date de publication et mention de la délivrance du brevet: **04.08.1993**
(21) Numéro de dépôt: 89403027.9
(22) Date de dépôt: 03.11.1989
(51) Int. Cl.: A61M 5/20, A61M 5/46

(54) **Guide de seringue avec réglage de la profondeur de pénétration de l'aiguille dans la peau**
Spritzenführung mit Einstellung der Einstichtiefe der Nadel in die Haut
Syringe guide with regulation of penetration depth into the skin

(30) Priorité: 03.11.1988 FR 8815375
(43) Date de publication de la demande: 06.06.1990
(73) Titulaire: Dalto, Tino, F-06000 Nice (FR); Laruelle, Claude, F-06270 Villeneuve-Loubet (FR)
(72) Inventeur: Dalto, Tino, F-06000 Nice (FR); Laruelle, Claude, F-06270 Villeneuve-Loubet (FR)
(74) Mandataire: Ramey, Daniel

(56) Documents cités:
- EP-A- 0 265 876
- DD-A- 250 467
- US-A- 2 874 694
- US-A- 2 959 170
- US-A- 4 194 505
- US-A- 4 629 454
- SOVIET INVENTIONS ILLUSTRATED Séction méchanique, Semaine 8717, no. de l'abrégé 120982, P34, 6 mai 1987, Derwent Publications Ltd., London, GB;

## Description

L'invention concerne un guide de seringue, permettant un réglage de la profondeur de pénétration de l'aiguille dans la peau, et permettant également de réaliser automatiquement l'enfoncement de l'aiguille dans la peau, de façon rapide et donc relativement indolore.

Le document DD-A-250 467 décrit un dispositif d'injection et de dosage, en particulier d'insuline, qui comprend une seringue contenant le produit à injecter et qui est munie d'une aiguille et d'un piston, une pièce tubulaire dans laquelle est montée la seringue et un corps cylindrique dans lequel est guidée cette pièce tubulaire, un ressort de poussée de la pièce tubulaire pour l'enfoncement de l'aiguille dans la peau d'un patient, et des moyens de réglage agissant sur le piston de la seringue pour le dosage d'une quantité de produit à injecter. Des moyens de blocage à levier pivotant et à ressort sont prévus sur le corps cylindrique pour bloquer la pièce tubulaire dans une position de compression du ressort et pour la libérer par action sur le levier pivotant. Le dispositif décrit dans ce document a une structure relativement complexe et est donc coûteux.

La présente invention propose un guide de seringue avec réglage de la profondeur de pénétration de l'aiguille dans la peau, comprenant un corps cylindrique dans lequel est montée la seringue et qui comporte une embase coulissante réglable en position sur le corps, par exemple au moyen d'une vis, une pièce cylindrique solidaire de la seringue et montée coulissante dans le corps et soumise à l'action d'un ressort de poussée, et des moyens de blocage prévus sur le corps pour bloquer la pièce dans une position où le ressort est comprimé et pour le libérer afin de permettre l'enfoncement de l'aiguille par détente de ce ressort, caractérisé en ce que les moyens de blocage comprennent une butée d'appui de la pièce, cette butée étant formée sur la paroi du corps, et une bague annulaire entourant le corps et comportant un poussoir à bille s'étendant à travers une ouverture du corps à l'opposé de la butée et un doigt transversal passant à travers la paroi du corps pour écarter la pièce de la butée.

Le dispositif selon l'invention permet ainsi de réaliser des piqûres automatiquement par simple action sur la bague annulaire des moyens de blocage qui maintiennent le ressort comprimé, dont la détente provoque l'enfoncement de l'aiguille sur une profondeur déterminée dans la peau du patient. Une personne peut donc se faire une piqûre à elle-même ou à une autre personne à l'aide d'une seule main.

Selon une autre caractéristique de l'invention, l'embase est de forme conique et comprend une face d'appui sur la peau, qui est relativement large.

Cette largeur relativement grande de la face d'appui de l'embase sur la peau permet un guidage de l'aiguille perpendiculairement à la peau. De plus, la présence de cette embase réduit l'appréhension du patient et permet à une personne inexpérimentée de faire des piqûres sans risques.

Dans la description qui suit, faite à titre d'exemple, on se réfère aux dessins annexés dans lesquels:
- la figure 1 est une vue schématique en coupe longitudinale d'une première forme de réalisation d'un guide de seringue selon l'invention;
- les figures 2 et 3 sont des vues de dessus de deux pièces de ce guide.

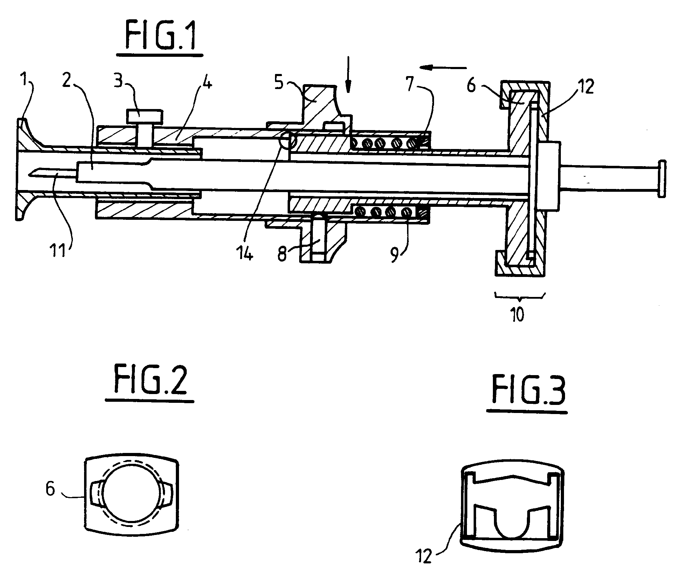

On se réfère d'abord à la figure 1 qui représente une forme de réalisation du guide de seringue selon l'invention.

Ce guide comprend essentiellement un corps fixe 4, réalisé en métal, et dans une extrémité duquel coulisse une embasse 1 de forme tronconique qui entoure avec jeu l'extrémité inférieure d'une seringue 2, montée à l'intérieur du corps 4. Une vis de réglage 3 traverse la paroi du corps 4 et permet d'immobiliser l'embase en position dans le corps 4.

L'autre extrémité de la seringue 2, comprenant des oreilles perpendiculaires à la direction longitudinale de la seringue, est rendue solidaire d'une pièce 6 montée coulissante dans l'autre extrémité du corps 4 et sollicitée par un ressort 9 prenant appui, d'une part sur un épaulement de la pièce 6, d'autre part sur un rebord annulaire du corps 4.

Une bague 5 est montée autour du corps 4 et comprend un poussoir 8 à bille qui s'étend transversalement à travers une ouverture du corps 4 et vient pousser la pièce coulissante 6 vers la paroi opposée du corps 4. Cette paroi opposée comprend une butée 14 sur laquelle vient s'appuyer l'extrémité de la pièce 6 qui, dans cette position, comprime le ressort 9. La bague 5 comprend par ailleurs un doigt transversal, sensiblement diamétralement opposé au poussoir 8, qui traverse la paroi du corps 4 et vient s'appliquer sur la pièce 6. Par pression sur la bague 5 au niveau de ce doigt, on dégage la pièce 6 de la butée 14 et le ressort 9 déplace alors la seringue vers la gauche en figure 1. La solidarisation de la pièce coulissante 6 et de la seringue 2 est réalisée au moyen d'une tête de fixation 10 constituée par un rebord annulaire de la pièce 6 et une plaque coulissante 12, qui maintiennent entre elles les oreilles transversales de la seringue et qui sont décrites plus en détail en référence aux figures 3 et 4.

Le dispositif selon l'invention est utilisé de la façon suivante.

Au moyen de la vis 3, on règle la position de l'embase 1 correspondant à la profondeur souhaitée de pénétration de l'aiguille dans la peau, puis on introduit la seringue 2 contenant un produit médicamenteux à l'intérieur de la pièce coulissante 6 et du corps 4, on bloque à l'aide de la plaque 12 les oreilles de la seringue sur le rebord annulaire de la pièce 6, et on tire la pièce 6 vers la droite de la figure 1 pour comprimer le ressort 9 et venir appliquer l'extrémité gauche de la pièce 6 sur la butée 14.

Il suffit ensuite de placer l'embase 1 sur la peau, à l'endroit souhaitée, en orientant l'ensemble du dispositif perpendiculairement à la peau, puis d'appuyer sur la bague 5 dans le sens voulu, pour libérer la pièce 6 et provoquer l'enfoncement de l'aiguille 11 dans la peau, sous l'effet de la détente du ressort 9. Il suffit ensuite d'enfoncer le piston de la seringue avec le pouce, pour l'injection du produit médicamenteux contenu dans la seringue.

Dans les figures 2 et 3, on a représenté plus en détail une forme de réalisation possible de la pièce 6 et de la plaque 12, respectivement.

Dans la figure 2, on voit que le rebord annulaire de la pièce 6 comprend deux renfoncements diamétralement opposés de réception des oreilles de la seringue 2. La plaque 12 représentée dans la figure 3 est montée transversalement coulissante sur ce rebord annulaire de la pièce 6 et peut occuper deux positions, l'une dans laquelle elle recouvre les oreilles de la seringue qui est alors solidarisée de la pièce 6, l'autre dans laquelle les oreilles et le corps de la seringue peuvent passer à travers une découpe appropriée de la plaque 12.

Il suffit donc de faire coulisser la plaque 12 d'une position à l'autre sur le rebord annulaire de la pièce 6, pour solidariser la seringue de la pièce 6 ou l'en désolidariser.

## Revendications

1. Guide de seringue avec réglage de la profondeur de pénétration de l'aiguille dans la peau, comprenant un corps cylindrique (4) dans lequel est montée la seringue (2) et qui comporte une embase coulissante (1) réglable en position sur le corps (4), par exemple au moyen d'une vis (3), une pièce cylindrique (6) solidaire de la seringue (2) et montée coulissante dans le corps (4) et soumise à l'action d'un ressort de poussée (9), et des moyens de blocage prévus sur le corps (4) pour bloquer la pièce (6) dans une position où le ressort (9) est comprimé et pour la libérer afin de permettre l'enfoncement de l'aiguille par détente de ce ressort, caractérisé en ce que les moyens de blocage comprennent une butée (14) d'appui de la pièce (6), cette butée (14) étant formée sur la paroi du corps (4), et une bague annulaire (5) entourant le corps (4) et comportant un poussoir à bille (8) s'étendant à travers une ouverture du corps (4) à l'opposé de la butée (14) et un doigt transversal passant à travers la paroi du corps (4) pour écarter la pièce (6) de la butée (14).

2. Guide selon la revendication 1, caractérisé en ce que l'embase (1) est de forme conique et comprend une face d'appui sur la peau, qui est relativement large.

3. Guide selon la revendication 1 ou 2, caractérisé en ce qu'une plaque (12) coulissante sur une extrémité de la pièce (6) permet de solidariser la seringue et la pièce (6) par recouvrement des oreilles latérales de la seringue et blocage sur un rebord de la pièce (6).

## Claims

1. A syringe guide where the depth of needle penetration through the skin is adjustable, the syringe guide comprising: a cylindrical body (4) in which the syringe (2) is mounted and including a sliding base (1) which is adjustable in position on the body (4), e.g. by means of a screw (3); a cylindrical part (6) fixed to the syringe (2), slidably mounted inside the body (4) and subjected to the action of a thrust spring (9); and locking means provided on the body (4) to lock the said part (6) in position when the spring (9) is compressed and to release it in order to enable the needle to be plunged into the skin under the action of the spring; the syringe guide being characterized in that the locking means comprise an abutment (14) for the said part (6), this abutment (14) being formed on the wall of the body (4), and an annular ring (5) encircling the body (4) and comprising a ball pusher (8) extending through an opening of the body (4) opposite the abutment (14) and a transverse finger passing through the wall of the body (4) in order to space the part (6) away from the abutment (14).

2. A guide according to claim 1, characterized in that the base (1) is conical in shape and includes a face for bearing against the skin, which face is relatively large.

3. A guide according to claim 1 or 2, characterized in that a plate (12) sliding on one end of the part (6) enables the syringe to be fixed to the part (6) by overlying lateral lugs of the syringe and locking them on a rim of the part (6).

## Patentansprüche

1. Spritzenführung mit Einstellung der Einstichtiefe der Nadel in die Haut, die aufweist: einen zylindrischen Körper (4), in welchem die Spritze (2) befestigt ist und der einen Stempel (1) aufweist, der bezüglich des Körpers (4), beispielsweise mittels einer Schraube (3), verstellbar ist, einen zylindrischen Teil (6), der mit der Spritze (2) verbunden ist und an dem Körper (4) unter der Wirkung einer Schubfeder (9) gleitend geführt ist, und an dem Körper (4) vorgesehene Blockiermittel, um den Teil (6) in einer Stellung zu blockieren, bei welcher die Feder (9) komprimiert ist, und um ihn zu lösen, sodaß das Eindringen der Spritze durch Entspannen dieser Feder ermöglicht wird, **dadurch gekennzeichnet, daß** die Blockiermittel einen Anschlag (14) zum Abstützen des Teils (6), wobei dieser Anschlag (14) an der Wand des Körpers (4) ausgebildet ist, und einen kreisförmigen Ring (5) aufweisen, welcher den Körper (4) umschließt und einen Drücker (8), der in einer seitlichen Öffnung des Körpers (4) liegt, die sich gegenüber dem Anschlag (14) befindet, und einen seitlichen Stift aufweist, der die Wand des Körpers (4) durchquert, um den Teil (6) von dem Anschlag (14) zu lösen.

2. Führung nach Anspruch 1, **dadurch gekennzeichnet, daß** der Stempel (1) konisch geformt ist und eine relativ große Abstützfläche für die Haut aufweist.

3. Führung nach Anspruch 1 oder 2, **dadurch gekennzeichnet, daß** eine an einem Ende des Teils (6) gleitende Platte (12) durch Übergriff auf seitliche Griffe der Spritze und durch Blockierung an einem Anschlag des Teils (6) das Verbinden der Spritze und des Teils (6) ermöglicht.
